# EUROPÄISCHE PATENTSCHRIFT

(11) **EP 1 140 031 B1**
(45) Veröffentlichungstag und Bekanntmachung des Hinweises auf die Patenterteilung: **22.10.2003**
(21) Anmeldenummer: 99967009.4
(22) Anmeldetag: 23.12.1999
(51) Int. Cl.: A61K 9/20, A61K 31/215

(54) **KONTROLLIERT FREISETZENDE PHARMAZEUTISCHE ZUSAMMENSETZUNG MIT TILIDINMESYLAT ALS WIRKSTOFF**
SUSTAINED-RELEASE PHARMACEUTICAL PREPARATION CONTAINING TILIDINE MESYLATE AS ACTIVE INGREDIENT
COMPOSITION PHARMACEUTIQUE A LIBERATION CONTROLEE CONTENANT COMME PRINCIPE ACTIF DU MESYLATE DE TILIDINE

(30) Priorität: 23.12.1998 DE 19859636
(43) Veröffentlichungstag der Anmeldung: 10.10.2001
(73) Patentinhaber: HEXAL AG, D-83607 Holzkirchen (DE)
(72) Erfinder: Hirsch, Richard, 39179 Barleben (DE); Wesseling, Martin, 39179 Barleben (DE); Strüngmann, Thomas, 83607 Holzkirchen (DE)
(74) Vertreter: Winter, Brandl & Partner
(86) Internationale Anmeldenummer: EP9910381
(87) Internationale Veröffentlichungsnummer: WO00038656

(56) Entgegenhaltungen:
- EP-A- 0 864 325
- WO-A-94/10129
- WO-A-99/55662

## Beschreibung

Die Erfindung betrifft eine kontrolliert freisetzende, feste, oral zu verabreichende, pharmazeutische Zusammensetzung, welche Tilidinmesylat als Wirkstoff enthält.

Tilidin [2-(Dimethylamino)-1-phenyl-3-cyclohexen-1-carboxylsäure-ethylester] stellt ein synthetisches Opioid aus der Gruppe der morphinartig wirkenden Analgetika dar. Es wird, ebenso wie dessen Salze, zur Behandlung von starken und sehr starken akuten und chronischen Schmerzen bei Traumen, postoperativen Zuständen, Knochen- und Gelenkserkrankungen, Schmerzen im Bereich der Brustorgane, Neuritis, Neuralgie, Tumorschmerzen, Abdominalspasmen, schmerzhaften Entzündungen, posttraumatischen Schmerzen und Schmerzen bei diagnostischen und therapeutischen Eingriffen eingesetzt.

Tilidin sowie dessen Salze hemmen die Erregerübertragung polysynaptischer Bahnen des nozirezeptiven Systems. Die Wirkung wird durch an den Neuronen befindlichen Opiatrezeptoren vermittelt, die auch als Bindungsstellen für natürlich vorkommende Peptide, sogenannte Enkephaline, dienen.

Tilidin sowie dessen Salze haben nur geringe analgetische Wirkung. Erst durch die Verstoffwechselung in der Leber entstehen die eigentlich wirksamen Metaboliten Nortilidin und Bisnortilidin. Die beiden Metaboliten werden als partielle Morphinantagonisten eingestuft, wobei sie dem Morphin in seinen pharmakokinetischen Eigenschaften sehr ähnlich sind.

Tilidin und viele seiner pharmazeutisch akzeptablen Salze, wie z.B. Tilidinhydrochlorid (Tilidin-HCl) lassen sich nur sehr schwer bzw. gar nicht als feste Darreichungsformen herstellen, da die Stabilität von Tilidin sehr gering ist. Tilidin in Verbindung mit festen Hilfsstoffen zersetzt sich sehr schnell bei der Herstellung und bei der Lagerung, was sich durch Verfärbungen bemerkbar macht. Zudem erweisen sich viele Salze des Tilidins als sehr hygroskopisch und damit schwer zu verarbeiten.

Das in der Patentschrift EP 0 665 830 B1 beschriebene Tilidindihydrogenorthophosphat stellt auf Grund seiner Stabilität das üblicherweise verwendete Salz von Tilidin für die Herstellung von festen Arzneiformen dar. Allerdings stellt die Herstellung dieses Salzes hohe Anforderungen an die Sicherheit, da ganz bestimmte Bedingungen eingehalten werden müssen, um kritische Situationen zu vermeiden.

Es wurde nun gefunden, daß Tilidinmesylat eine ausreichend hohe Stabilität aufweist und somit sehr gut zur Herstellung von festen Arzneiformen geeignet ist, weil es in fester Form, das heißt in Verbindung mit festen Hilfsstoffen praktisch keine Zersetzung erleidet. Aufgrund der hohen Stabilität wird zudem eine leichte Verarbeitung gewährleistet, da keine besonderen Anforderungen an die Klimatisierung der Arbeitsräume und an den Korrosionsschutz der verwendeten Apparaturen und Werkzeuge notwendig sind. Die Herstellung des Tilidinmesylats kann ohne besondere Sicherheitsvorkehrungen unter Verwendung der üblichen Laborgerätschaften erfolgen.

Obwohl die Therapie mit schnell freisetzenden Wirkstoffen durch häufige und dabei regelmäßige Einnahme des Wirkstoffes versucht, einen andauernden therapeutisch wirksamen Blutplasmaspiegel des Wirkstoffes zu erhalten, treten doch aufgrund der sofortigen Absorption, der systemischen Ausscheidung und der hepatischen Metabolisierung des Wirkstoffes große Schwankungen des Blutplasmaspiegels auf. Dadurch kann die Effektivität des Wirkstoffes stark beeinträchtigt werden.

Die Aufgabe der vorliegenden Erfindung ist es nun, eine feste, oral zu verabreichende, kontrolliert freisetzende pharmazeutische Zusammensetzung, welche Tilidinmesylat als Wirkstoff enthält, bereitzustellen, so daß die Patienten-Compliance durch eine geringere Einnahmehäufigkeit verbessert und die Effektivität des Wirkstoffes optimiert wird.

Unter "kontrolliert freisetzend" wird hier eine Freisetzungsrate des Wirkstoffes verstanden, bei der ein therapeutisch wirksamer Blutplasmaspiegel über einen Zeitraum von mindestens 8-12 Stunden und gegebenenfalls bis zu 24 Stunden erreicht wird. Ein therapeutisch wirksamer Blutplasmaspiegel liegt insbesondere zwischen 30 und 50 ng/ml Nortilidin.

Es wurde nun gefunden, daß mittels der erfindungsgemäßen Zusammensetzung über einen Zeitraum von mindestens 8 bis 12 Stunden und gegebenenfalls bis zu 24 Stunden ein gleichmäßiger Blutplasmaspiegel gewährleistet werden kann. Die Einnahmehäufigkeit kann somit auf eine ein oder zwei Mal tägliche Gabe reduziert werden.

Die erfindungsgemäße Zusammensetzung enthält pro Dosierungseinheit eine analgetisch wirksame Menge an Tilidinmesylat, die einer Menge von 50-500 mg Tilidinhydrochlorid (Tilidin-HCl) entspricht, wobei die bevorzugten Dosiseinheiten eine Menge Tilidinmesylat enthalten, die 50 mg, 100 mg, 150 mg und 200 mg Tilidin-HCl entsprechen.

Die erfindungsgemäße Zusammensetzung kann in Form von Granulaten, Pellets, Spheroiden und/ oder Extrudaten vorliegen. Diese können entweder in Kapseln oder Sachets abgefüllt oder zu Tabletten verpreßt werden. Auch kann gegebenenfalls der Wirkstoff und mögliche Hilfsstoffe direkt tablettiert werden.

Der in der erfindungsgemäßen Zusammensetzung verwendete Wirkstoff Tilidinmesylat kann in eine Matrix, die ein gelbildendes Matrixmaterial umfaßt, eingebettet sein. Diese Matrix gewährleistet die kontrollierte Freisetzung von Tilidinmesylat über einen Zeitraum von mindestens 8 bis 12 Stunden und gegebenenfalls bis zu 24 Stunden (matrixkontrolliert).

Geeignete matrixbildende Materialien:
a) Hydrophile oder hydrophobe Polymere, wie z.B. Gummis, Celluloseether, Celluloseester, Acrylharze , Materialen, die auf Proteinen basieren, Nylon, Polyvinylchlorid, Stärke und/ oder Polyvinylpyrrolidon. Geeignete wasserlösliche Polymere sind u.a. Polyvinylpyrrolidon, Hydroxypropylcellulose, Hydroxypropylmethylcellulose, Hydroxyethylcellulose, Hydroxymethylcellulose, Poly(vinylalkohole), Alginate, Polydextrose, Carboxymethylen, hydrierte Hydroxyalkylcellulose und/ oder Hydroxypropylmethylcelluloseether. Als wasserunlösliche Polymere können Polyvinylchlorid, Ethylcellulose, Methylcellulose, Carboxymethylcellulose (teilweise wasserlöslich, je nach mittleren Substitutionsgrad), Celluloseacetate, Celluloseacetatphthalate, Ethylenvinylalkohol, Alginsäure und/ oder deren Derivate, Acrylsäure- und/ oder Methacrylsäure-Copolymere, Methylmethacrylat-Copolymere, Ethoxyethylmethacrylat-Copolymere, Cyanoethylmethacrylate, Aminoalkylmethacrylat-Copolymere, Poly(acrylsäure), Poly(methacrylsäure), Methacrylsäurealkylamid-Copolymere, Poly(methylmethacrylate), Poly(methacrylsäureanhydride), Methylmethacrylate, Polymethacrylate, Poly(methylmethacrylat)-Copolymer, Polyacrylamide, Aminoalkylmethacrylat-Copolymere und/ oder Glycidylmethacrylat-Copolymere verwendet werden. Die erfindungsgemäße Zusammensetzung kann 1-90 % (Gewichtsprozent) von einem oder mehreren der hydrophilen oder hydrophoben Polymeren als Matrix enthalten.
b) Verdauliche, langkettige (C₈ -C₅₀, insbesondere C₁₂ -C₄₀), substituierte oder unsubstituierte Kohlenwasserstoffe, wie z.B. Fettsäuren, Fettalkohole (Lauryl-, Myrestyl-, Stearyl-, Cetostearyl-, Ceryl- oder Cetylalkohol), Glycerinester von Fettsäuren (Witepsol, Glycerinmonostearat), Mineral- und Pflanzenöle (hydriertes Rizinusöl) und/ oder Wachse (Paraffinwachse, Silikonwachse, Bienenwachse, Rizinuswachse, Carnaubawachse und/ oder Glycowachse). Die Kohlenwasserstoffe, die einen Schmelzpunkt zwischen 25 °C und 90 °C aufweisen, sind besonders geeignet. Die bevorzugten langkettigen Kohlenwasserstoffe stellen die Fettalkohole dar. Die erfindungsgemäße Zusammensetzung kann mindestens einen der verdaulichen, langkettigen Kohlenwasserstoffe enthalten, wobei der Gehalt bei bis zu 60 % (Gewichtsprozent) bezogen auf die Matrix liegen kann.
c) Polyalkylenglykole, wobei die erfindungsgemäße Zusammensetzung bis zu 60 % (Gewichtsprozent) eines oder mehrerer Polyalkylenglykole bezogen auf die Matrix enthalten kann.

Die bevorzugte erfindungsgemäße Matrixform kann den Wirkstoff Tilidinmesylat in einer gelbildenden Matrix aus z.B. Hydroxymethylcellulose, Hydroxyethylcellulose, Hydroxypropylcellulose, Hydroxypropylmethylcellulose, Alginat und/ oder Polyacrylsäure, insbesondere Hydroxypropylmethylcellulose, enthalten. Das Polymer hydratisiert und bildet eine gelartige Schicht, d.h. eine Hydrogelmatrix, welche langsam per Diffusion und Erosion den Wirkstoff kontrolliert freisetzt.

Bei einer weiteren erfindungsgemäße Matrixform kann der Wirkstoff Tilidinmesylat mit bekannten wasserlöslichen Hilfsstoffen und fettähnlichen Stoffen kombiniert werden. Als lipophile Stoffe können abbaubare Mono-, Di- und Triglyceride (Glycerinmonostearat, Glycerinmonooleat, Glycerintripalmitat), aber auch erodierbare Fettalkohole (Lauryl-, Myristyl-, Stearyl-, Cetyl- und/ oder Cerylalkohol) mit einem Schmelzpunkt von 30-80 °C verwendet werden. Die Wirkstoffabgabe erfolgt durch Diffusion und durch enzymatischen Abbau der lipophilen Stoffe. Das Einbetten des Wirkstoffes in die Matrix erfolgt durch Schmelzen, Sprüherstarren, Sprühtrocknen, Granulieren oder Direkttablettieren.

Eine weitere geeignete kontrolliert freisetzende Matrixform kann neben dem Wirkstoff Tilidinmesylat, bekannte, wasserlösliche Hilfsstoffe, welche, genauso wie der Wirkstoff, in ein Gerüst, gebildet aus wasserunlöslichen, unverdaulichen Hilfsstoffen, eingebettet sind, enthalten. Durch Herauslösen der löslichen Bestandteile entstehen Poren, durch die der Wirkstoff nach außen diffundiert. Als gerüstbildende Substanzen können Polymere wie Polyvinylchlorid, Polyethylen, Polyamid, Silicone, Ethylcellulose und/ oder Methacryl-Acrylat-Copolymere eingesetzt werden. Das Wirkstoff-Hilfsstoff-Gemisch wird entweder direkt oder nach dem Feuchtgranulieren mit organischen Lösungsmitteln bzw. Bindemittellösungen zu Tabletten verpreßt oder in Pelletform in Kapseln gefüllt.

Eine derartige kontrolliert freisetzende Matrix kann aus einer oder mehreren Alkylcellulosen und einem oder mehreren C₁₂ -C₃₆ aliphatischen Alkoholen sowie wahlweise mindestens einem Polyalkylenglykol bestehen. Vorzugsweise wird eine C₁ - C₆ Alkylcellulose, insbesondere Ethylcellulose, verwendet. Der Gehalt an Alkylcellulosen in der Matrix kann 1-20 % (Gewichtsprozent), insbesondere 2-15 % (Gewichtsprozent) betragen.

Brauchbare aliphatische Alkohole stellen Lauryl-, Myrestyl-, Stearyl-, Cetostearyl-, Ceryl- und/ oder Cetylalkohol dar. Der Gehalt an aliphatischen Alkoholen in der Matrix kann 5-30 % (Gewichtsprozent), insbesondere 10-25 % (Gewichtsprozent) betragen.

Bevorzugt verwendet wird Polyethylenglykol als Polyalkylenglykol-Komponente.

Andere pharmazeutisch geeignete Hilfsstoffe, welche nach dem Stand der Technik üblich sind, wie z.B. Verdünnungsmittel, Schmiermittel, Bindemittel, Granulierungshilfsmittel, Farbstoffe, Aromastoffe, Detergenzien, Puffer, Antihaftmittel und/ oder Gleitmittel, können zudem in der kontrolliert freisetzenden Matrix enthalten sein.

Die in eine kontrolliert freisetzende Matrix eingebettete erfindungsgemäße Zusammensetzung kann noch mit einem bekannten, pharmazeutisch geeigneten, nicht kontrolliert freisetzenden Überzugsmittel filmbeschichtet werden. Bevorzugt werden wässerige Filmüberzüge, wie sie beispielsweise unter dem Markennamen Opadry® erhältlich sind.

Eine weitere Ausführungsform der Erfindung kann aus einer Initialdosis und einer verzögert freisetzenden Komponente bestehen. Die Initialdosis beinhaltet Tilidinmesylat als Pulver, Granulat und/oder Pellets, jeweils neben fakultativen Hilfsstoffen. Das in der Initialdosis beinhaltete Tilidinmesylat wird sofort nach der Einnahme freigesetzt. Der therapeutisch wirksame Blutplasmaspiegel wird mittels dieser Initialdosis sehr schnell erreicht, so daß kurz nach der Einnahme eine therapeutische Wirkung zu verzeichnen ist. Die verzögert freisetzende Komponente enthält Tilidinmesylat als Granulat und/oder Pellets, jeweils neben fakultativen Hilfsstoffen. Diese verzögert freisetzende Komponente ist für die Aufrechterhaltung des therapeutisch wirksamen Blutplasmaspiegels über mehrere Stunden verantwortlich. Dem Patienten kann somit über mehrere Stunden ein gleichmäßig hoher Blutplasmaspiegel garantiert werden. Das Granulat oder die Pellets können retardierende Hilfsstoffe enthalten, welche eine kontrolliert freisetzende Matrix bilden. Als geeignete Stoffe, welche eine kontrolliert freisetzende Matrix bilden, können die oben erwähnten Matrixbildner verwendet werden.

Die erfindungsgemäße Zubereitung kann in Form einer Zweischichttablette vorliegen. Die erste Schicht stellt die Initialdosis dar, welche aus Pulver und gegebenenfalls Hilfsstoffen, dem oben beschriebenen Granulat und/ oder Pellets gepreßt wird. Die zweite Schicht beinhaltet die oben beschriebene verzögert freisetzende Komponente, welche aus dem entsprechenden Granulat und/ oder Pellets gepreßt wird.

Bei der Initialdosis kann der Gehalt an Tilidinmesylat 5-30 Gew.-% des Gesamttilidinmesylat-Gehaltes betragen.

Zur Herstellung der oben genannten Dosierungsformen können die nach dem Stand der Technik bekannten pharmazeutischen Hilfsstoffe, wie Tablettenbinder, Füllstoffe, Konservierungsmittel, Tablettensprengmittel, Fließregulierungsrnittel, Weichmacher, Netzmittel, Dispergiermittel, Emulgator, Retardierungsmittel, Antioxidantien und/ oder sonstige bekannte Träger- und Verdünnungsmittel verwendet werden.

Die erfindungsgemäße Zusammensetzung kann wahlweise neben Tilidinmesylat den Opiatantagonisten Naloxon enthalten. Naloxon verhindert den Mißbrauch von Heroinabhängigen und verringert das Risiko der Abhängigkeit. Zudem kann die Zusammensetzung noch Citronensäure, Ascorbinsäure und/oder deren Derivate, Schwefeldioxid, Natriumsulfit, Natriumbisulfit und/oder Tocopherol sowie dessen wasser- und fettlöslichen Derivate, beispielsweise solche, die unter dem Markennamen Tocofersolan® erhältlich sind, oder Tocopherolacetat, Sulfite, Bisulfite und/ oder Hydrogensulfite von Alkali-, Erdalkalie und/oder anderen Metallen, PHB-Ester, BHA, BHT, Gallate sowie niedere Fettsäuren, Fruchtsäuren, Phosphorsäuren, Sorbin- und/ oder Benzoesäure sowie deren Salze, Ester, Derivate und/ oder isomere Verbindungen, Ascorbylpalmitat, Lecithin, ein- und mehrfach hydroxylierte Benzolabkömmlinge, Äthylendiamintetraessigsäure und/ oder deren Salze, Citraconsäure, Cystein, L-Cystein, Conidendrine, Diethylcarbonate, Methylendioxyphenole, Kephaline, β,β'-Dithiopropionsäure, Biphenyl und/ oder andere Phenylderivate enthalten, was zu einer Stabilisierung der Zusammensetzung dienen kann.

Die Freisetzungsrate der erfindungsgemäßen Zusammensetzung in vitro beträgt 5-50%, vorzugsweise 10-40% (Gewichtsprozent) Tilidinmesylat nach einer Stunde, 10-75%, vorzugsweise 20-50% (Gewichtsprozent) Tilidinmesylat nach zwei Stunden, 20-95%, vorzugsweise 30-80% (Gewichtsprozent) Tilidinmesylat nach vier Stunden, 40-100%, vorzugsweise 50-100% (Gewichtsprozent) Tilidinmesylat nach acht Stunden, mehr als 50%, vorzugsweise mehr als 60% (Gewichtsprozent) Tilidinmesylat nach zwölf Stunden, mehr als 70%, vorzugsweise mehr als 80% (Gewichtsprozent) Tilidinmesylat nach achtzehn Stunden und mehr als 80%, vorzugsweise mehr als 90% (Gewichtsprozent) Tilidinmesylat nach vierundzwanzig Stunden, vorzugsweise bestimmt nach USP, Basket Methode, in 1000 ml 0,1 M HCl.

Des weiteren wird bei der erfindungsgemäßen Zusammensetzung nach acht Stunden mindestens 70-95 % (Gewichtsprozent) Tilidinmesylat, nach zehn Stunden mindestens 77-97 % (Gewichtsprozent) Tilidinmesylat und nach zwölf Stunden mindestens 80-100 % Tilidinmesylat in vivo absorbiert.

Die Erfindung wird durch nachstehende Beispiele näher erläutert.

### Beispiel 1:

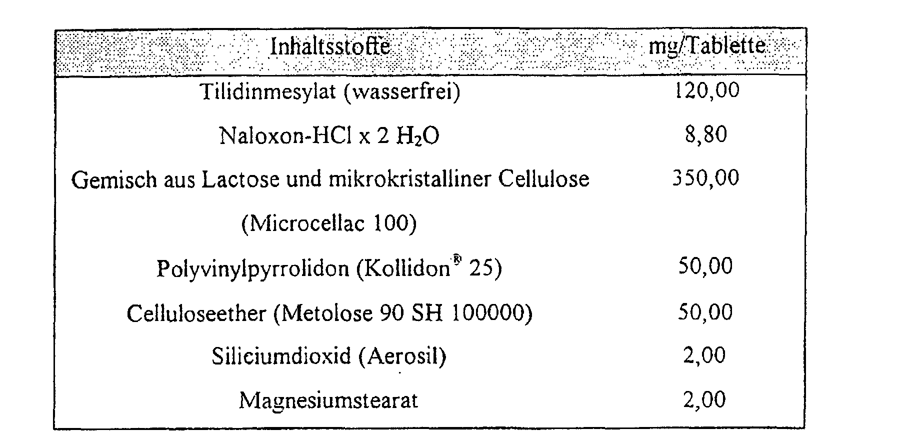

Die Komponenten 1 bis 6 werden gesiebt und innig vermischt. Magnesiumstearat wird ebenfalls gesiebt und mit dem Gemisch der Komponenten 1 bis 6 vermischt. Die Zusammensetzung wird direkt tablettiert. Der Tablettenkern (Gesamtgewicht 582,8 mg ) wird nach der Verpressung mit einem wässerigem Film (Opadry®) überzogen. Die Filmdicke ist dabei variabel.

### Beispiel 2:

Die folgenden Stoffe werden zur Herstellung von 1000 Tabletten [253,64 mg Gesamtgewicht, 59,64 mg Tilidinmesylat (entspricht 50 mg Tilidin-HCl bzw. 44,12 mg Tilidn)] verwendet.

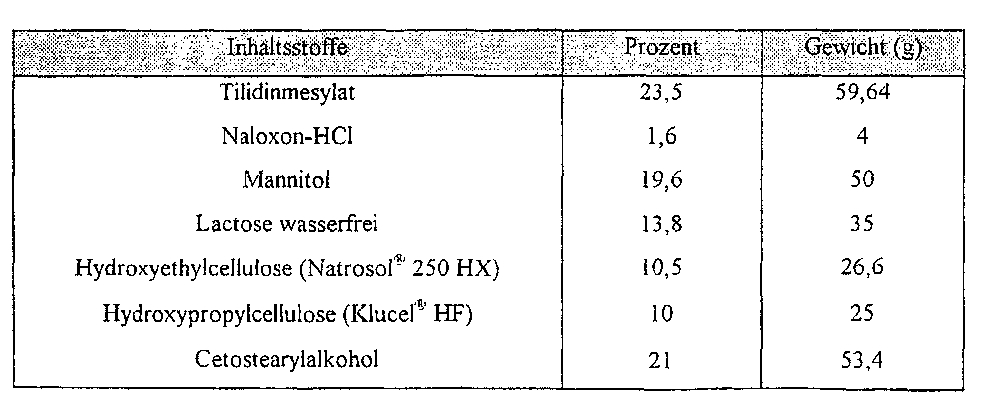

Tilidinmesylat, Naloxon-HCI, Mannitol, Lactose, Hydroxyethylcellulose und Hydroxypropylcellulose (15g als Bindemittel) werden trocken gemischt. Die Mischung wird anschließend mit 439 g Wasser angefeuchtet, bis eine feuchte Granuliermasse erhalten wird. Die angefeuchtete Mischung wird dann in einem Fluid Bed Dryer (FBD) bei 60 °C leicht angetrocknet, granuliert und durch ein Sieb mit einer Maschengröße von 12 gesiebt. Das Granulat wird anschließend vollständig in einem FBD bei 60 °C getrocknet, regranuliert und durch ein 1,25 mm Sieb (Maschengröße 16) gesiebt. Zu dem warmen Tilidinmesylatgranulat wird geschmolzener Cetylstearylalkohol hinzugefügt und durchgemischt. Die Mischung wird im Luftstrom abgekühlt, regranuliert und durch ein 1,25 mm Sieb gesiebt. Die restliche Hydroxypropylcellulose (10 g) wird zu der Mischung hinzugefügt und mit dem Granulat gemischt, bis das Granulat einen ausreichend dicken Überzug aus Hydroxypropylcellulose aufweist. Anschließend wird das Granulat zu Tabletten verpreßt. Die Tabletten können bei Bedarf noch mit Standardbeschichtungen überzogen werden.

### Beispiel 3:

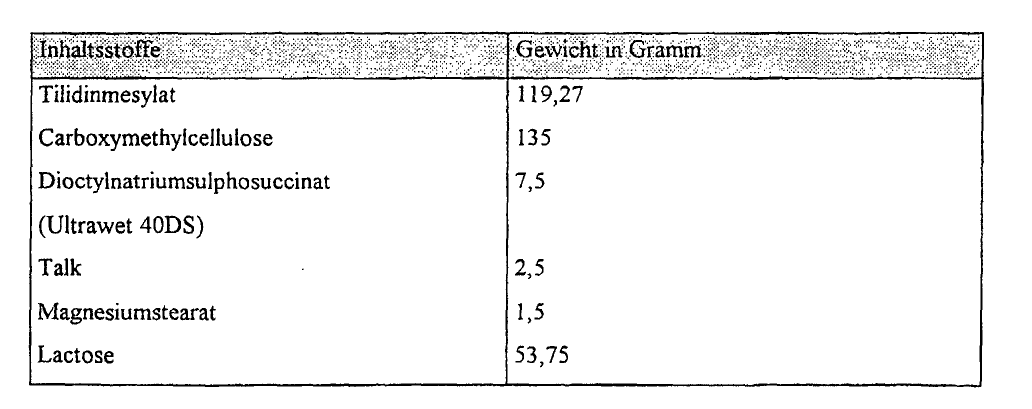

Dioctylnatriumsulphosuccinat wird in Isopropanol gelöst. Carboxymethylcellulose wird mit dieser Lösung gemischt bis die Mischung homogen ist. Die Mischung wird dann granuliert und durch ein Sieb mit einer Maschengröße von 16 gesiebt. Das Granulat wird im Luftstrom getrocknet bis das gesamte Isopropanol verdampft ist. Tilidinmesylat und Lactose werden hinzugefügt und gemischt. Anschließend wird Magnesiumstearat und Talk beigemengt und solange gemischt, bis eine homogene Mischung vorliegt. Im Anschluß wird die Mischung regranuliert und durch ein Sieb mit einer Maschengröße von 16 gesiebt. Das erhaltene Granulat wird zu Tabletten verpreßt bzw. in Kapseln oder Sachets abgefüllt. Die Menge pro Kapsel, Tablette oder Sachet an Tilidinmesylat beträgt 119,27 mg, was einer Menge an Tilidin-HCl von 100 mg und einer Menge an Tilidin von 88,24 mg entspricht. Es werden 1000 Stück mit je einem Gesamtgewicht von 319,52 mg hergestellt.

### Beispiel 4:

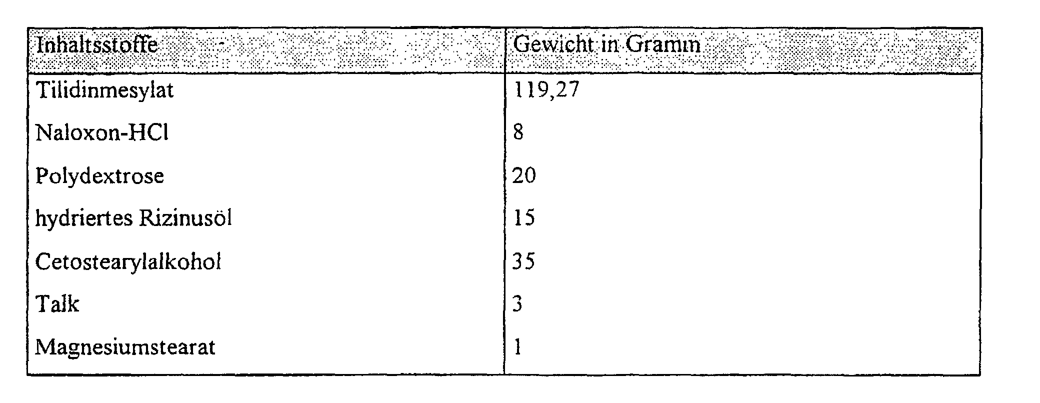

Tilidinmesylat, Naloxon-HCl und das hydrierte Rizinusöl werden mit Polydextrose granuliert und das Granulat wird durch ein 1,25 mm Sieb gesiebt und bei 60 °C in einem FBD getrocknet. Zu diesem Granulat wird geschmolzener Cetostearylalkohol hinzugefügt. Diese Mischung wird im Luftstrom abgekühlt und erneut durch ein 1,25 mm Sieb gesiebt. Talk und Magnesiumstearat werden mit dem Granulat vermischt und dann entweder zu Tabletten verpreßt oder in Kapseln und Sachets abgefüllt. Dieser Ansatz ergibt 1000 Einheiten mit einem Gesamtgewicht von 201,07 mg/Einheit. Jede Tablette, Kapsel oder Sachet enthält 119,27 mg Tilidinmesylat, was einer Tilidin-HCl-Menge von 100 mg und einer Tilidinmenge von 88,24 mg entspricht.

### Beispiel 5:

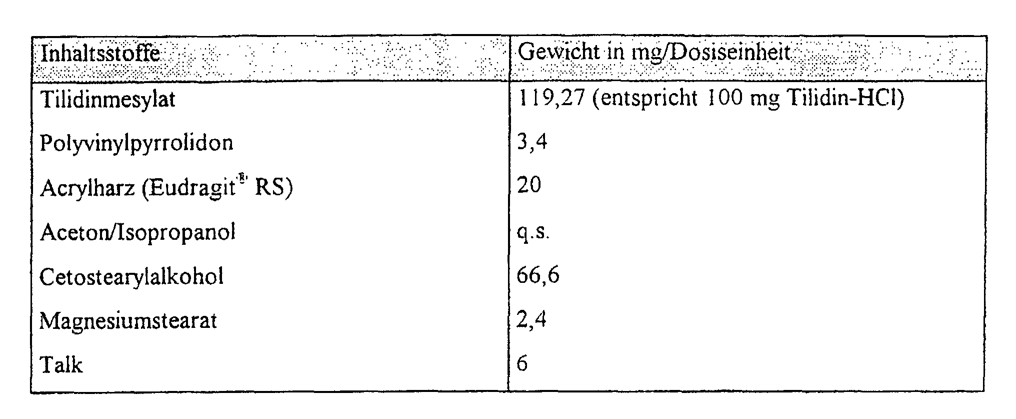

Tilidinmesylat und Polyvinylpyrrolidon (PVP) werden zügig in einer entsprechenden Apparatur gemischt. Eudragit wird in Aceton/Isopropanol (50:50) gelöst (Granulierungsfluid). Dieses Granulierungsfluid wird nun langsam während des Mischvorganges von Tilidinmesylat und PVP hinzu gegeben bis eine feuchte Granulierungsmasse entsteht. Das entstandene Granulat wird anschließend getrocknet und durch ein Sieb mit einer Maschengröße von 12 gesiebt. Der bei einer Temperatur von 60-70 °C geschmolzene Cetostearylalkohol wird zu dem noch warmen Granulat gegeben und gemischt. Nach dem Abkühlen wird das Granulat durch ein 1,7 mm Sieb gesiebt. Anschließend wird der Talk und das Magnesiumstearat beigemengt und untergemischt. Das Granulat wird entweder zu Tabletten verpreßt oder in Kapseln oder Sachets gefüllt.

### Beispiel 6:

Natürlicher Gummi (SMR 20 NR, 1g) wird in Toluol (100 ml) unter Rückfluß während 24 Stunden gelöst. Die daraus resultierende leicht gelbe Lösung wird abgekühlt und der geringe Anteil an ungelöstem Gummi sedimentiert. Dieser Sedimentationsschritt kann durch Zentrifugieren beschleunigt werden. 12 ml dieser klaren Lösung werden mit 10 ml Toluol verdünnt und für den weiteren Herstellungsschritt verwendet.
Tilidinmesylat (3,05g), Magnesiumstearat (0,58 g), Cellulosepuder (0,44 g) und Ethylenvinylacetat-Copolymer (Vinnapas RE 530Z, 5,81 g) werden gemischt und homogenisiert. Dieses Gemisch wird mit der oben beschriebenen Lösung verrieben. Eine feuchte Suspension wird erhalten. Diese wird im Vakuum während häufigerem Verreiben getrocknet. Die so erhaltene Matrix kann direkt zur Tablettierung verwendet werden.

### Beispiel 7:

238,54 g Tilidinmesylat und 16 g Näloxon-HCl werden mit 40 g Ethylcellulose und 25 g Polyvinylpyrrolidon vermischt. Danach werden 140 g Lactose und 203 g Talk hinzugefügt, mit einer ausreichenden Menge an Alkohol angefeuchtet und granuliert sowie anschließend getrocknet. Das erhaltene Granulat wird entweder zu Tabletten verpreßt oder in Kapseln gefüllt. Eine derart hergestellte Dosiseinheit enthält 238,54 mg Tilidinmesylat (entspricht einer Menge von 200 mg Tilidin-HCI und einer Menge von 176,5 mg Tilidin).

### Beispiel 8:

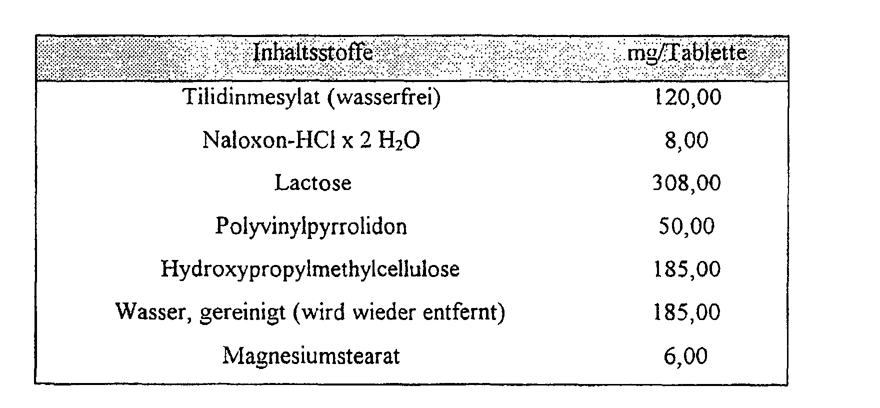

Es wurde wie in Beispiel 7 beschrieben ein Granulat hergestellt, das nach dem Trocknen zu Tabletten verpreßt wurde.

### Beispiel 9:

### Zweischichttablette:

### Initialdosis:

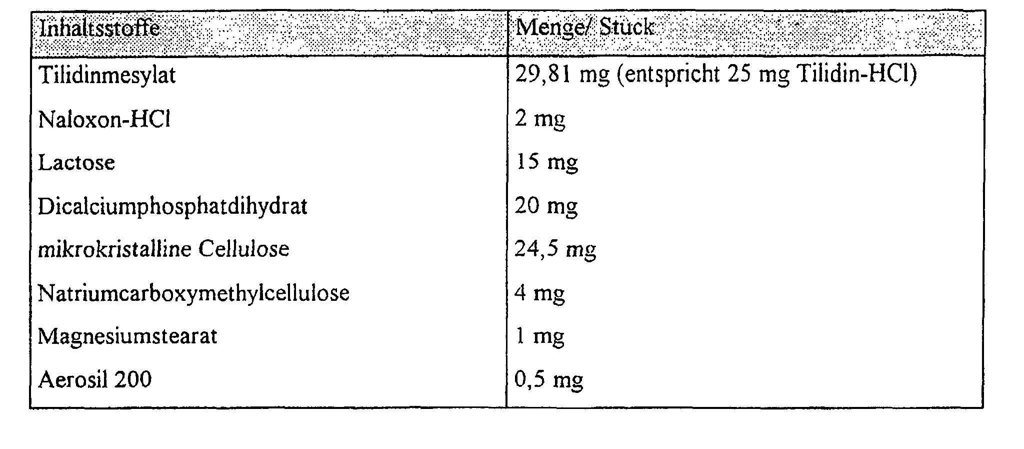

### Retarddosis:

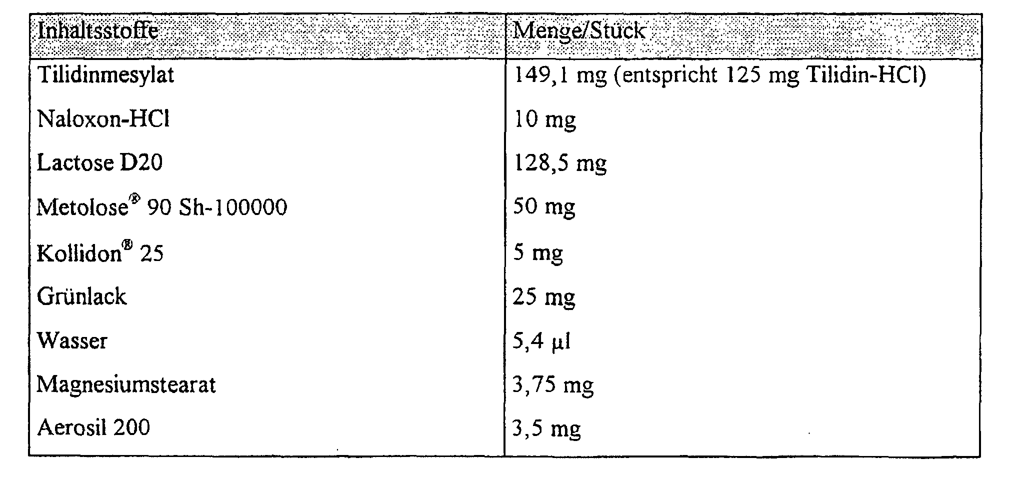

### Herstellung des Retardgranulats:

Kollidon® wird in Wasser gelöst und der Grünlack darin dispergiert. Tilidinmesylat, Naloxon-HCl Lactose und Metolose® werden in einem Wirbelschichtgranulator vorgelegt und mit der vorher hergestellten Lösung granuliert. Zu dem erhaltenen Granulat wird Magnesiumstearat und Aerosil dazugegeben und durch ein 1,0 mm Zwangssieb gegeben und im Containermischer homogenisiert.

### Herstellung der Initialdosis:

Die gesamten Inhaltsstoffe der Initialdosis werden durch ein 0,8 mm Zwangssieb gegeben und im Containermischer homogenisiert.

Das Retardgranulat wird mit der Initialdosis auf einer geeigneten Rundlauftablettenmaschine zu Zweischichttabletten verpreßt.

## Patentansprüche

1. Feste, orale pharmazeutische Zusammensetzung mit kontrolliert freisetzender Matrix enthaltend Tilidinmesylat als Wirkstoff, **dadurch gekennzeichnet, daß** die kontrolliert freisetzende Matrix ein gelbildendes Matrixmaterial umfaßt.

2. Feste, orale pharmazeutische Zusammensetzung mit kontrolliert freisetzender Matrix nach Anspruch 1, **gekennzeichnet durch** einen Gehalt an Tilidinmesylat, der 50-500 mg Tilidin-HCl entspricht, wobei die bevorzugten Dosiseinheiten eine Menge Tilidinmesylat enthalten, die 50 mg, 100 mg, 150 mg und 200 mg Tilidin-HCl entspricht.

3. Feste, orale pharmazeutische Zusammensetzung mit kontrolliert freisetzender Matrix nach Anspruch 1 oder 2, **dadurch gekennzeichnet, daß** die kontrolliert freisetzende Matrix hydrophile und/oder hydrophobe Polymere, insbesondere Gummi, Celluloseether, Celluloseester, Materialen, die auf Proteinen basieren, Nylon, Polyvinylchlorid, Stärke, Polyvinylpyrrolidon, Alginate, Polydextrose, Carboxymethylen, Acrylharze Poly(vinylalkohole), Ethylenvinylalkohol, Alginsäure und/oder deren Derivate und/oder Fettsäuren, Fettalkohole, Glycerinester von Fettsäuren, Mineral- und Pflanzenöle, Wachse und/oder Polyalkylenglykole als Retardierungsmittel enthält.

4. Feste, orale pharmazeutische Zusammensetzung mit kontrolliert freisetzender Matrix nach einem der vorangegangenen Ansprüche in der Form von Pulver, Granulat, Pellets, Spheroiden und/ oder Extrudaten, welche in Kapseln oder Sachets abgefüllt oder zu Tabletten verpreßt werden können.

5. Feste, orale pharmazeutische Zusammensetzung mit kontrolliert freisetzender Matrix nach einem der vorangegangenen Ansprüche, welche folgende Komponenten umfaßt oder aus ihnen besteht:
i) eine Wirkstoffinitialdosis, die eine therapeutisch wirksame Menge an Tilidinmesylat als aktiven Bestandteil neben fakultativen Hilfsstoffen enthält.
ii) eine verzögert freisetzende Komponente, welche eine therapeutisch wirksame Menge an Tilidinmesylat als aktiven Bestandteil neben fakultativen Hilfsstoffen enthält.

6. Feste, orale pharmazeutische Zusammensetzung mit kontrolliert freisetzender Matrix nach Anspruch 5 in Form einer Zweischichttablette, wobei die erste Schicht aus der Initialdosis und die zweite Schicht aus der verzögert freisetzenden Komponente besteht.

7. Feste, orale pharmazeutische Zusammensetzung mit kontrollierter Freisetzung nach einem der vorangegangenen Ansprüche, **gekennzeichnet durch** Tablettenbinder, Füllstoffe, Konservierungsmittel, Tablettensprengmittel, Fließregulierungsmittel, Schmiermittel, Weichmacher, Netzmittel, Dispergiermittel, Emulgator, Retardierungsmittel Bindemittel, Granulierungshilfsmittel, Farbstofffe, Aromastoffe, Detergenzien, Puffer, Antihaftmittel, Gleitmittel, Antioxidantien und/ oder sonstige bekannte Träger- und Verdünnungsmittel als Hilfsstoffe.

8. Feste, orale pharmazeutische Zusammensetzung mit kontrollierter Freisetzung nach einem der vorangegangenen Ansprüche, **gekennzeichnet durch** die Kombination des Wirkstoffes Tilidinmesylat mit Naloxon und/ oder dessen pharmazeutisch unbedenklichen Salzen.

## Claims

1. Solid pharmaceutical composition for oral administration having a controlled-release matrix, containing tilidine mesylate as an active ingredient, **characterized in that** the controlled-release matrix comprises a gel-forming matrix material.

2. Solid pharmaceutical composition for oral administration having a controlled-release matrix according to claim. 1, **characterized by** a tilidine mesylate content corresponding to 50-500 mg of tilidine-HCl, the preferred dosage units containing an amount of tilidine mesylate corresponding to 50 mg, 100 mg, 150 mg and 200 mg of tilidine-HCl.

3. Solid pharmaceutical composition for oral administration having a controlled-release matrix according to claim 1 or 2, **characterized in that** the controlled-release matrix contains, as retarding agent, hydrophilic and/or hydrophobic polymers, in particular gum, cellulose ethers, cellulose esters, protein-based materials, nylon, polyvinyl chloride, starch, polyvinyl pyrrolidone, alginates, polydextrose, carboxymethylene, acrylic resins, poly(vinyl alcohols), ethylene vinyl alcohol, alginic acid and/or derivatives thereof, and/or fatty acids, fatty alcohols, fatty acid glycerol esters, mineral and vegetable oils, waxes and/or polyalkylene glycols.

4. Solid pharmaceutical composition for oral administration having a controlled-release matrix according to any one of the preceding claims in the form of powder, granulate, pellets, spheroids and/or extrudates, which can be filled into capsules or sachets or pressed to tablets.

5. Solid pharmaceutical composition for oral administration having a controlled-release matrix according to any one of the preceding claims, comprising or consisting of the following components:
i) an initial dose of active ingredient containing a therapeutically effective amount of tilidine mesylate as an active principle along with optional additives;
ii) a delayed-release component containing a therapeutically effective amount of tilidine mesylate as an active principle along with optional additives.

6. Solid pharmaceutical composition for oral administration having a controlled-release matrix according to claim 5 in the form of a two-layer tablet, wherein the first layer consists of the initial dose and the second layer consists of the delayed-release component.

7. Solid pharmaceutical controlled-release composition for oral administration according to any one of the preceding claims, **characterized by** a content of tablet binders, fillers, preservatives, tablet disintegrants, flow regulating agents, lubricants, plasticizers, wetting agents, dispersing agents, emulsifiers, retarding agents, binders, granulation aids, colourants, flavouring agents, detergents, buffers, antiblocking agents, slip agents, antioxidants and/or other known carrier materials and diluents as additives.

8. Solid pharmaceutical controlled-release composition for oral administration according to any one of the preceding claims, **characterized by** a combination of the active ingredient tilidine mesylate with naloxone and/or pharmaceutically acceptable salts thereof.

## Revendications

1. Composition pharmaceutique orale solide à matrice à libération contrôlée contenant du mésylate de tilidine comme principe actif, **caractérisée en ce que** la matrice à libération contrôlée comprend un matériau de matrice gélifiant.

2. Composition pharmaceutique orale solide à matrice à libération contrôlée selon la revendication 1 **caractérisée par** une teneur en mésylate de tilidine qui correspond à 50-500 mg de tilidine-HCl, où les unités de dose préférées contiennent une quantité de mésylate de tilidine qui correspond à 50 mg, 100 mg, 150 mg et 200 mg de tilidine-HCl.

3. Composition pharmaceutique orale solide à matrice à libération contrôlée selon la revendication 1 ou 2 **caractérisée en ce que** la matrice à libération contrôlée contient des polymères hydrophiles et/ou hydrophobes, en particulier du caoutchouc, des éthers cellulosiques, des esters cellulosiques, des matériaux qui sont à base de protéines, du Nylon, du poly(chlorure de vinyle), de l'amidon, de la polyvinylpyrrolidone, des alginates, du polydextrose, du carboxyméthylène, des résines acryliques, des poly(alcools vinyliques), de l'alcool éthylène-vinylique, de l'acide alginique et/ou ses dérivés et/ou des acides gras, des alcools gras, des esters de glycérine et d'acides gras, des huiles minérales et végétales, des cires et/ou des polyalkylèneglycols comme agents de retard.

4. Composition pharmaceutique orale solide à matrice à libération contrôlée selon l'une des revendications précédentes sous forme de poudre, de granulés, de pastilles, de sphéroïdes et/ou d'extrudats, qui peuvent être introduits dans des capsules ou des sachets ou compressés en comprimés.

5. Composition pharmaceutique orale solide à matrice à libération contrôlée selon l'une des revendications précédentes qui comprend les composants suivants ou qui consiste en les composants suivants :
i) une dose initiale de principe actif qui contient une quantité thérapeutiquement efficace de mésylate de tilidine comme constituant actif, outre des adjuvants facultatifs ;
ii) un composant à libération retardée qui contient une quantité thérapeutiquement efficace de mésylate de tilidine comme constituant actif, outre des adjuvants facultatifs.

6. Composition pharmaceutique orale solide à matrice à libération contrôlée selon la revendication 5 sous forme d'un comprimé à deux couches, où la première couche consiste en la dose initiale et la deuxième couche consiste en le composant à libération retardée.

7. Composition pharmaceutique orale solide à libération contrôlée selon l'une des revendications précédentes **caractérisée par** des liants pour comprimés, des charges, des conservateurs, des agents de délitement pour comprimés, des agents régulant l'écoulement, des lubrifiants, des plastifiants, des agents mouillants, des dispersants, des émulsifiants, des agents de retard, des liants, des adjuvants de granulation, des colorants, des arômes, des détergents, des tampons, des agents anti-adhésion, des agents de glissement, des antioxydants et/ou d'autres supports et diluants connus comme adjuvants.

8. Composition pharmaceutique orale solide à libération contrôlée selon l'une des revendications précédentes **caractérisée par** la combinaison du principe actif mésylate de tilidine avec la naloxone et/ou ses sels pharmaceutiquement acceptables.
